# EUROPEAN PATENT SPECIFICATION

(11) **EP 3 646 831 B1**
(45) Date of publication and mention of the grant of the patent: **01.11.2023**
(21) Application number: 17928595.2
(22) Date of filing: 12.10.2017
(51) Int. Cl.: A61F 13/49, A61F 13/496, A61F 13/514, A61F 13/539, A61F 13/513

(54) **ABSORBENT ARTICLE**
ABSORBIERENDER ARTIKEL
ARTICLE ABSORBANT

(43) Date of publication of application: 06.05.2020
(73) Proprietor: Unicharm Corporation, Shikokuchuo-shi, Ehime 799-0111 (JP)
(72) Inventor: FUJII, Keishi, Kanonji-shi Kagawa 769-1602 (JP); UEDA, Masumi, Kanonji-shi Kagawa 769-1602 (JP)
(74) Representative: Dolleymores
(86) International application number: PCT/JP2017/036996
(87) International publication number: WO 2019/073568

(56) References cited:
- EP-A1- 1 541 098
- EP-A1- 2 186 495
- WO-A1-2014/192981
- WO-A1-2017/002206
- WO-A2-96/31179
- JP-A- H03 280 951
- JP-A- 2014 050 749
- JP-A- 2015 066 197
- JP-A- 2015 107 223

## Description

### Technical Field

The present invention relates to an absorbent article.

### Background

A disposable diaper and the like are known as an absorbent article that absorbs excrements such as urine. For example, PTL 1 discloses forming a plurality of slits 8 penetrating in the thickness direction to prevent stuffiness inside a tape-type disposable diaper 1 and a pull-on disposable diaper 30.

### Citation List

### Patent Literature

[PTL 1] Japanese Patent Application Publication No. 2002-65731
EP 2186495 A1 relates to an absorbent article with tear resistance that allows the wearer's skin to be seen through the absorbent article: in one embodiment, belt members of the absorbent article are provided with a plurality of vent holes extending through the belt members in a thickness direction thereof.

### Summary

### Technical Problem

With the disposable diapers 1, 30, indicated in PTL 1 in the natural state, however, due to the contraction of a waist elastic member 7, there is a possibility that a wearer or a person putting on the disposable diapers 1, 30 cannot visually identify slits 8, and good air permeability cannot be recognized from the outside.

This invention has been made in view of the above issues, and an object is to make through holes of an absorbent article easy to be visually identified by a wearer of the absorbent article or a person putting on the absorbent article to easily recognize good air permeability.

### Solution to Problem

The present invention provides the absorbent article of independent claim 1. The dependent claims specify preferred but optional features.

Amain aspect of the invention to achieve the above is an absorbent article including an up-down direction, a right-left direction, and a front-rear direction, which intersect with each other, including: a front-side waist part; and a rear-side waist part, at least one of the front-side waist part and the rear-side waist part including an elastic member that expands and contracts in the right-left direction and holes penetrating in the front-rear direction, of the front-side waist part and the rear-side waist part, a waist part on one side with a smaller contractile force in the right-left direction includes more of the holes than a waist part on another side with a greater contractile force in the right-left direction.

Other features of this invention will become clear from descriptions in this specification and attached drawings.

### Advantageous Effects of Invention

According to the present invention, because the possibility that through holes will be hidden with creases formed with contraction of the elastic members can be decreased, the through holes of the absorbent article in the natural state can be easily visually identified, and good air permeability can be easily realized.

### Brief Description of Drawings

[Fig. 1] Fig. 1A is a schematic front view of a diaper 1 in a natural state seen from a front side as an example of an absorbent article of the present embodiment. Fig. 1B is a schematic rear view of a diaper 1 in a natural state seen from a rear side.
[Fig. 2] Fig. 2 is a plan view of a diaper 1 in an open stretched state seen from a skin side.
[Fig. 3] Fig. 3 is a sectional view along line III-III in Fig. 2.
[Fig. 4] Fig. 4 is a sectional view along line IV-IV in Fig. 2.
[Fig. 5] Fig. 5A is a schematic view of a section along Va-Va of a front-side waist part 20 in Fig. 1A. Fig. 5B is a schematic view of a section along Vb-Vb of a rear-side waist part 30 in Fig. 1B.
[Fig. 6] Fig. 6 is a schematic side view of a diaper 1 seen from a one end side in the right-left direction.
[Fig. 7] Fig. 7 is a plan view of a diaper 1 in an extended state that is open seen from a skin side according to another embodiment.

### Description of Embodiments

At least below matters will become clear from descriptions in this specification and attached drawings.

An absorbent article including an up-down direction, a right-left direction, and a front-rear direction, which intersect with each other, including: a front-side waist part; and a rear-side waist part, at least one of the front-side waist part and the rear-side waist part including an elastic member that expands and contracts in the right-left direction and holes penetrating in the front-rear direction, of the front-side waist part and the rear-side waist part, a waist part on one side with a smaller contractile force in the right-left direction includes more of the holes than a waist part on another side with a greater contractile force in the right-left direction.

According to such an absorbent article, because the possibility that through holes will be hidden with creases formed with contraction of the elastic members can be decreased, the through holes of the absorbent article in the natural state can be easily visually identified, and good air permeability can be easily realized.

With an absorbent article, wherein preferably each of the front-side waist part and the rear-side waist part includes the holes.

According to such an absorbent article, air permeability in both the front and the back can be improved.

With an absorbent article, wherein preferably the waist part on one side is the rear-side waist part.

According to such an absorbent article, the rear-side waist part including more holes can be easily distinguished.

With an absorbent article, wherein preferably a non-overlapping region, of the rear-side waist part, that does not overlap with the front-side waist part in the front-rear direction includes at least one of the holes.

According to such an absorbent article, even when the absorbent article is viewed from the front side, the hole(s) included in the rear-side waist part can be visually identified, and good air permeability can be realized by visual identification.

With an absorbent article, wherein preferably the rear-side waist part includes an overlapping region that overlaps with the front-side waist part in the front-rear direction, an interval between a first lower end elastic member positioned to a lowest side in the non-overlapping region and the elastic member adjacent to the first lower end elastic member in the upper side is greater than an interval between a second lower end elastic member positioned to a lowest side in the overlapping region and the elastic member adjacent to the second lower end elastic member in the upper side.

According to such an absorbent article, because the contractile force of the non-overlapping region can be made smaller, the possibility that the holes in the non-overlapping region will be covered with creases is decreased, and even when viewing the absorbent article from the front side, the holes in the rear-side waist part can be easily visually identified.

With an absorbent article, wherein preferably the rear-side waist part includes an overlapping region that overlaps with the front-side waist part in the front-rear direction, a tension of a first lower end elastic member positioned to a lowest side in the non-overlapping region is smaller than a tension of a second lower end elastic member positioned to a lowest side in the overlapping region.

According to such an absorbent article, because the contractile force in the non-overlapping region can be made smaller, the possibility that the holes in the non-overlapping region will be covered with creases is decreased, and even when viewing the absorbent article from the front side, the holes in the rear-side waist part can be easily visually identified.

With an absorbent article, wherein preferably the waist part on one side is the front-side waist part.

According to such an absorbent article, the front-side waist part including more holes can be easily distinguished.

With an absorbent article, wherein preferably an absorbent body is included, and a length of a region in which the waist part on one side overlaps with the absorbent body, in the up-down direction, is longer than a length of a region in which the waist part to the other side overlaps with the absorbent body.

According to such an absorbent article, the holes provided in the region overlapping the absorbent body with a relatively high rigidity in the up-down direction can easily maintain the open state, thus the holes of the waist part on one side can be more easily visually identified.

With an absorbent article, wherein an absorbent body is included, and a number of the holes in a region, of the waist part on one side, overlapping the absorbent body in the up-down direction is greater than a number of the holes in a region, of the waist part on one side, not overlapping the absorbent body in the up-down direction.

According to such an absorbent article, the holes provided in the region overlapping the absorbent body with a relatively high rigidity can easily maintain the open state, thus good air permeability can be easily visually identified.

With an absorbent article, wherein preferably the holes are provided in a region, of the waist part on one side, overlapping the absorbent body in the up-down direction, and the holes are not provided in a region, of the waist part on one side, not overlapping the absorbent body in the up-down direction.

According to such an absorbent article, the holes provided in the region overlapping the absorbent body with a relatively high rigidity can easily maintain the open state, thus good air permeability can be easily visually identified.

With an absorbent article, wherein preferably both end parts of the front-side waist part in the right-left direction and both end parts of the rear-side waist part in the right-left direction are joined to each other, and in a natural state, the front-side waist part and the rear-side waist part are in a protruding shape to the side of the waist part on one side.

According to such an absorbent article, the holes included in the waist part on one side can be more easily visually identified.

### === Disposable diaper of the present embodiment ===

### <<<Configuration of Disposable diaper 1»>

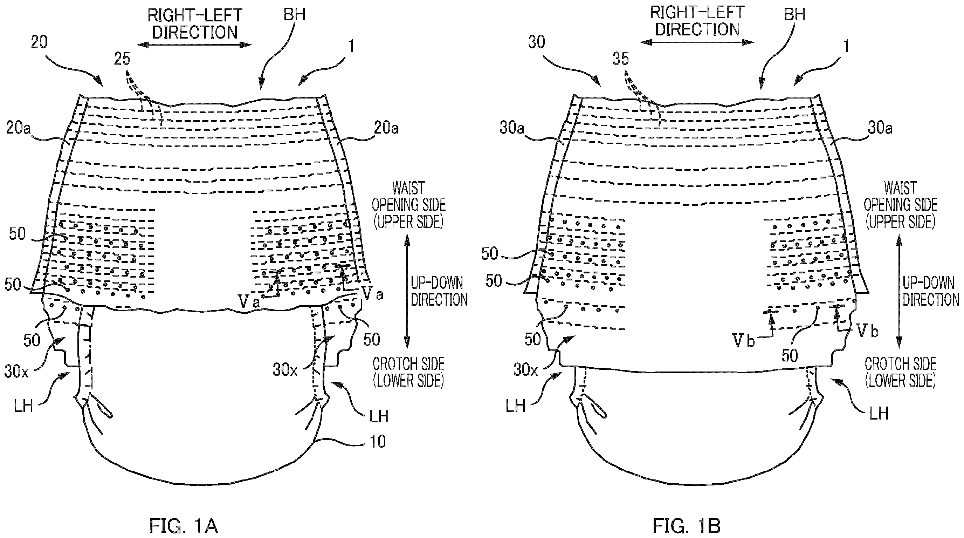

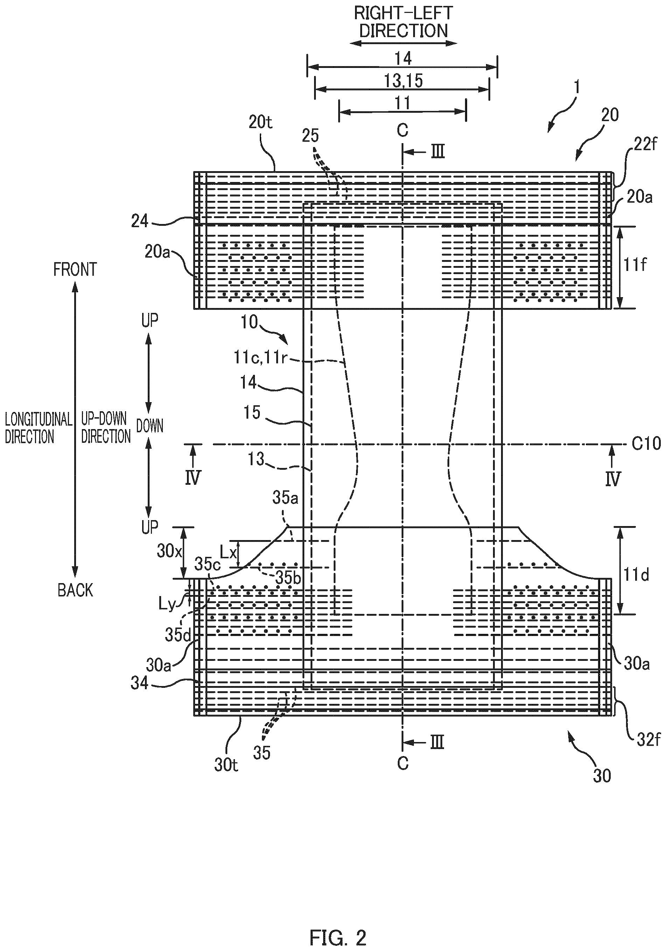

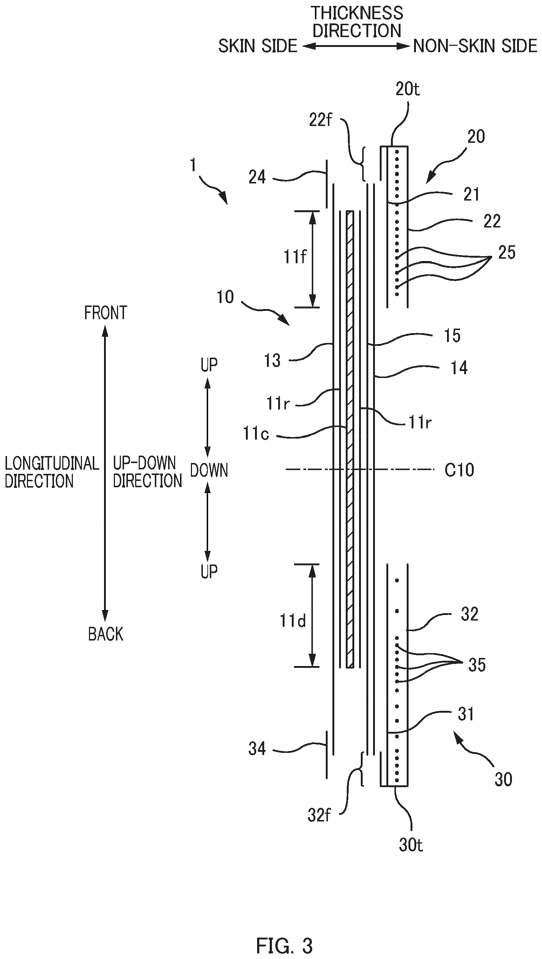

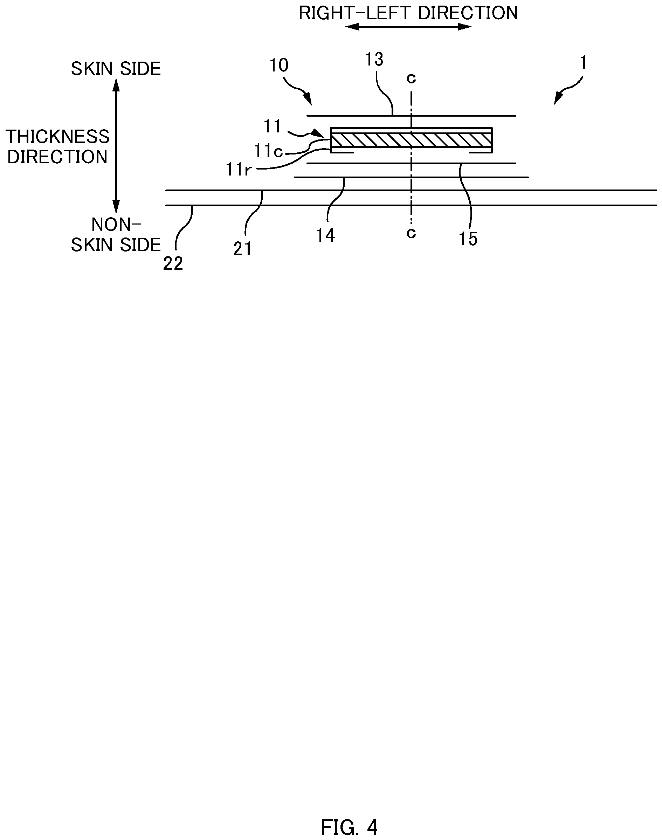

Fig. 1A is a schematic front view of a diaper 1 in a natural state seen from a front side as an example of an absorbent article of the present embodiment. Fig. 1B is a schematic rear view of a diaper 1 in a natural state seen from a rear side. Fig. 2 is a plan view of a diaper 1 in an open extended state seen from a skin side. Fig. 3 is a sectional view along line III-III in Fig. 2. Fig. 4 is a sectional view along line IV-IV in Fig. 2.

In the below description, the diaper 1 in a state in Fig. 1 (a natural state) includes an "up-down direction", a waist opening BH side of the diaper 1 in the up-down direction is referred to as an "upper side" and a crotch side is referred to as a "lower side". The diaper 1 includes a "right-left direction" that intersects with the up-down direction and a "front-rear direction" that intersects with the up-down direction and the right-left direction, and a front side in the front-rear direction is referred to also as "front" and a rear side to as "back". The front-rear direction is also referred to as a "thickness direction", and a side that comes in contact with a wearer is referred to as a "skin side", and an opposite side is referred to as a "non-skin side". A longitudinal direction of the diaper 1 in the state in Fig. 2 (an open state and an extended state) is referred to also as a "longitudinal direction", one side in the longitudinal direction is referred to also as "front", and another side is also referred to as "back". The end on one side in the longitudinal direction and the end on the other side are referred to as "upper side", and the approximately-central part C10 side in the longitudinal direction is referred to also as a "lower side". Line C-C in Fig. 2 and the like shows a center in the right-left direction.

A "natural state" of the diaper 1 is defined as below. After the diaper 1 that is wrapped as a product is taken out of a package, a front-side waist part 20 and a rear-side waist part 30 are pulled to both outer sides in the right-left direction, and the front-side waist part 20 and the rear-side waist part 30 are extended to a length that matches or is close to a dimension of each independent member. This extended state is maintained for 15 seconds, and then the extended state of the diaper 1 is released and placed on a plane such as a desk. The state of the diaper 1 that has been maintained for 5 minutes in the state placed on the plane is the natural state of the diaper 1. An "open state" is a state where joints of side end parts 20a of the front-side waist part 20 and side end parts 30a of the rear-side waist part 30 are separated and opened to open the entire diaper 1 in a plane. The "extended state" is a state where the diaper 1 (the front-side waist part 20 and the rear-side waist part 30) has been extended until there are no creases, and specifically is a state where the dimension of the members (the front-side waist part 20 and the rear-side waist part 30) configuring the diaper 1 are extended to a length to match or come close to the dimension of the independent members.

The disposable diaper 1 (hereafter referred to as "diaper 1") of this embodiment is namely a three-piece type pull-on diaper that is mainly to be worn by babies and infants. The disposable diaper 1 includes an absorbent main body 10 to be arranged in a crotch of a user, the front-side waist part 20 to cover the front of a wearer, and the rear-side waist part 30 to cover the back of a wearer. Each of the front-side waist part 20 and the rear-side waist part 30 is also referred to as a "waist part".

As shown in Fig. 2, with the diaper 1 in the open state, the front-side waist part 20 and the rear-side waist part 30 are arranged in parallel with an interval in the longitudinal direction, an absorbent main body 10 is placed over and between these waist parts 20, 30, end parts in a vertical direction of the absorbent main body 10 and each waist part 20, 30 is joined and fixed with such as an adhesive, thus the diaper 1 is in an approximately H shape in planar view. From the open state in the approximately H shape, the absorbent main body 10 is folded in two with an approximately central part C10 in the longitudinal direction as a folding position, and in the folded state, both end parts in the right-left direction of the front-side waist part 20 and the rear-side waist part 30 opposing each other, namely side end parts (also referred to as "end parts") 20a of the front-side waist part 20 and side end parts (also referred to as "end parts") 30a of the rear-side waist part 30 are joined by such as welding. Then, the front-side waist part 20 and the rear-side waist part 30 are joined annularly, to form a pull-on diaper 1 provided with a waist opening BH and a pair of leg openings LH, LH as shown in Fig. 1A and Fig. 1B.

As shown in Fig. 2, Fig. 3, and Fig. 4, the absorbent main body 10 is an approximately rectangular shape in planar view, and the longitudinal direction of the absorbent main body 10 is arranged along the up-down direction. Further, the absorbent main body 10 includes an absorbent body 11, a top sheet 13 that covers the absorbent body 11 from the skin side, an exterior sheet 14 that covers the absorbent body 11 from the non-skin side, and a back sheet 15.

The absorbent body 11 includes a liquid-absorbent absorbent core 11c that absorbs excrements such as urine, and a liquid permeable core-wrapping sheet 11r such as a tissue paper to coat an outer peripheral surface of the absorbent core 11c. The absorbent core 11c is a molded body that is formed by molding a predetermined liquid permeable material into an approximately sand-glass shape in planar view as an example of a predetermined shape. As a liquid absorbent material, liquid absorbent fibers such as pulp fibers and liquid absorbent particles such as superabsorbent polymers (namely SAP) can be given.

The top sheet 13 is a liquid permeable sheet such as a nonwoven fabric of a planar size that protrudes from the absorbent body 11 in the up-down direction and the right-left direction. The back sheet 15 is a sheet with a planar size that protrudes from the absorbent body 11 in the up-down direction and the right-left direction, and is a liquid impermeable leak-proof sheet. A nonwoven fabric exterior sheet 14 is provided to a non-skin side of the back sheet 15. Leg gathers (not shown) that expand and contract in the up-down direction of each of the outer sides in the right-left direction of the absorbent body 11 of the absorbent main body 10 may be provided. Further, to decrease the possibility of excrements leaking from outer sides in the right-left direction of the absorbent main body 10, barrier cuffs (not shown) may be provided as leak-proof wall parts in end parts of the absorbent main body 10 in the right-left direction.

As shown in Fig. 3, the front-side waist part 20 is made by placing and joining the skin side sheet 21 and the non-skin side sheet 22 on the skin side in the thickness direction in order, and the rear-side waist part 30 is made by placing and joining the skin side sheet 31 and the non-skin side sheet 32 on the skin side in the thickness direction in order. The skin side sheet 21, the non-skin side sheet 22, the skin side sheet 31, and the non-skin side sheet 32 are all made of a sheet member approximately rectangular in planar view made of such as spunbond or SMS (spunbond/meltblown/spunbond) nonwoven fabric. In this embodiment, SMS nonwoven fabric is used as the skin side sheets 21, 31, and spunbond nonwoven fabric is used as the non-skin side sheets 22, 32. The spunbond nonwoven fabric may be made of thermoplastic resin single fiber such as polypropylene (PP) or polyethylene (PE) or conjugate fiber such as a PP and PE core-sheath structure.

The skin side sheet 21, the non-skin side sheet 22, the skin side sheet 31, the non-skin side sheet 32 are joined with an adhesive such as a hot-melt adhesive to form the front-side waist part 20 and the rear-side waist part 30.

As shown in Fig. 3, to improve the texture and durability, in an upper side end part of the front-side waist part 20, the non-skin side sheet 22 is folded downward to the skin side, with a front-side upper end 20t that is an upper end of the front-side waist part 20 as the starting point, to form the folded-back section 22f. Similarly, in an upper part of the rear-side waist part 30, the non-skin side sheet 32 is folded downward to the skin side, with a rear-side upper end 30t that is an upper end of the rear-side waist part 30 as the starting point, to form the folded-back section 32f.

The front-side waist part 20 includes a sheet member 24 arranged to cover, from the skin side, a lower end part of the folded-back section 22f to an upper end part of the front of the absorbent main body 10. Similarly, the rear-side waist part 30 includes a sheet member 34 arranged to cover, from the skin side, a lower end part of the folded-back section 32f to an upper end part to the back of the absorbent main body 10. The sheet members 24, 34 are rectangular sheet members made of such as nonwoven fabric. The end parts of the absorbent main body 10 in the up-down direction can be prevented from directly contacting the skin of a wearer with these sheet members 24, 34, and the texture in the waist becomes satisfactory when wearing the diaper. Further, the sheet members 24, 34, can increase the strength of the upper-side end parts of the front-side waist part 20 and the rear-side waist part 30 in the up-down direction.

The front-side waist part 20 and the rear-side waist part 30 are provided with a plurality of approximately circular holes 50 that penetrate from the skin side to the non-skin side with a predetermined interval in between in the up-down direction and the left-right direction. The details of the holes 50 will be described later.

A plurality of elastic members 25, 25 ... such as elastic strings are arranged along the right-left direction between the skin side sheet 21 and the non-skin side sheet 22 of the front-side waist part 20. The elastic members 25 are joined fixedly with such as an adhesive to the skin side sheet 21 and the non-skin side sheet 22 in a state extended in the right-left direction. The plurality of elastic members 25, 25 ... are arranged aligned with an interval in between in the up-down direction.

Similarly, a plurality of elastic members 35, 35 ... such as elastic strings are arranged along the right-left direction between the skin side sheet 31 and the non-skin side sheet 32 of the rear-side waist part 30. The elastic members 35 are joined fixedly with such as an adhesive to the skin side sheet 31 and the non-skin side sheet 32 in a state extended in the right-left direction. The plurality of elastic members 35, 35 ... are arranged aligned with an interval in between in the up-down direction.

The elastic members 25, 35 add elasticity in the right-left direction in respect to the front-side waist part 20 and the rear-side waist part 30, in order to form a plurality of creases to the front-side waist part 20 and the rear-side waist part 30. Many of the creases formed in the front-side waist part 20 and the rear-side waist part 30 shorten each of the sheets 21, 22, 31, 32 in the right-left direction, and the sheets are shaped along the up-down direction.

Regions of the elastic members 25, 35 that overlap with the absorbent body 11 near the central part in the right-left direction are made non-continuous, so that a stretching force does not have an effect. In this way, contraction in the right-left direction that acts on the absorbent body 11 is suppressed, and the absorbent body 11 can be easily maintained in an approximately flat state, thus being able to suppress such as leakage of excrements.

### «<Holes 50»>

The holes 50 will be described below. The plurality of holes 50 provided to the front-side waist part 20 and the rear-side waist part 30 each penetrate through the skin side sheets 21, 31 and the non-skin side sheets 22, 32, and improve air permeability from the skin side to the non-skin side, when wearing the diaper 1. Each of the holes 50 in the front-side waist part 20 and the rear-side waist part 30 are approximately circular and approximately the same size. As shown in Fig. 2, in the front-side waist part 20, the plurality of the holes 50 are arranged staggered between the elastic members 25 adjacent in the up-down direction in regions 11f which are to the outer sides of the absorbent main body 10 to the right and left and which overlap with the absorbent body 11 in the up-down direction. In the rear-side waist part 30, the plurality of the holes 50 are arranged staggered between the elastic members 35 adjacent in the up-down direction in regions 11d which are to the outer sides of the absorbent main body 10 in the right and left direction and which overlap with the absorbent body 11 in the up-down direction. Each of the holes 50 does not cut the elastic members 25, 35, and the elastic members 25, 35 are preferably not made non-continuous with the holes 50. The length of the region 11f and the region 11d in the up-down direction of the diaper 1 is approximately the same.

A waist part that has a smaller contractile force in the right-left direction (a waist part on one side) has more holes 50 than a waist part that has a greater contractile force in the right-left direction (a waist part on another side) . In this embodiment, the rear-side waist part (a waist part on one side) 30 with a smaller contractile force in the right-left direction has more holes 50 than the front-side waist part (a waist part on another side) 20 that has a greater contractile force in the right-left direction.

The structure of the holes 50 of the diaper 1 will be specifically described below. "Contractile force" is a force that maintains a contraction state of the front-side waist part 20 or the rear-side waist part 30 when in a natural state. More specifically, in the case where the front-side waist part 20 or the rear-side waist part 30 is each divided into a right half and a left half in an arbitrary position of the diaper 1 in the right-left direction, a force that acts mutually between the right half and the left half is the contractile force. The greater this contractile force, the more contracted the diaper is in the natural state, and the more the creases are formed.

In the case that the length of the front-side waist part 20 and the rear-side waist part 30 in the extended state are the same, in order to decide which waist part has greater contractile force, the one that contracts more in the natural state may be decided as the waist part with the greater contractile force. More specifically, the joints between side end parts 20a of the front-side waist part 20 and side end parts 30a of the rear-side waist part 30 of the pull-on type diaper 1 as shown in Fig. 1A and Fig. 1B are divided, and the front-side waist part 20 and the rear-side waist part 30 are each pulled to both outer sides in the right-left direction, and extended such that the front-side waist part 20 and the rear-side waist part 30 each match or are close to the length of dimensions of each single member. After this extended state is continued for 15 seconds, the extended state is released and the diaper is placed on a flat surface such as a table, and after 5 minutes has passed while being placed on this surface, it is decided which of the front-side waist part 20 and the rear-side waist part 30 has contracted more. With the diaper 1 of this embodiment, because the front-side waist part 20 has contracted more than the rear-side waist part 30, the front-side waist part 20 has a greater contractile force than the rear-side waist part 30, and the rear-side waist part 30 has a smaller contractile force than the front-side waist part 20. Thus, more holes 50 are provided to the rear-side waist part 30 than the front-side waist part 20. The rear-side waist part 30 is also referred to as a "waist part on one side" and the front-side waist part 20 is also referred to as a "waist part on another/other side".

When comparing the size of the contractile force of a tape-type disposable diaper, in the case where only one of the front-side waist part or the rear-side waist part includes the elastic members, of course the waist part with the elastic members can be decided as having a greater contractile force.

Further, "the waist part with the greater contractile force" can also be referred to as a waist part with a smaller value obtained by dividing "a difference between the length in the extended state in the right-left direction and the length in the natural state in the right-left direction" with "the length of the extended state in the right-left direction".

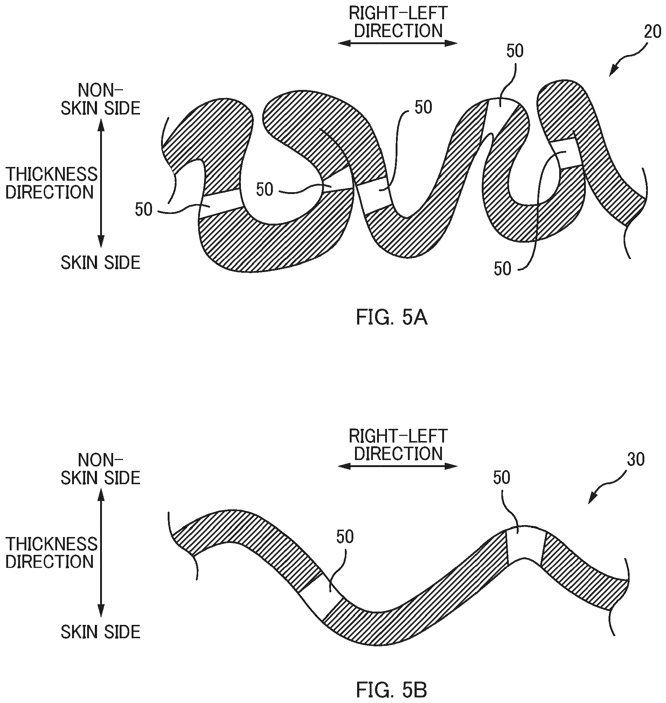

Fig. 5A is a schematic view of a section along Va-Va of the front-side waist part 20 in Fig. 1A, and Fig. 5B is a schematic view of a section along Vb-Vb of a rear-side waist part 30 in Fig. 1B. In Fig. 5A and Fig. 5B, the skin side sheet 21 and the non-skin side sheet 22, and the skin side sheet 31 and the non-skin side sheet 32 are shown in an integrated manner, and a cross section is shown in a region hatched with lines drawn diagonally to the left lower direction. The section along Va-Va of the front-side waist part 20 in Fig. 1A shown in Fig. 5A and the section along Vb-Vb of the rear-side waist part 30 in Fig. 1B shown in Fig. 5B show a region (unit region) in which the length in the right-left direction is approximately the same.

As shown in Fig. 5A and Fig. 5B, each of the front-side waist part 20 and the rear-side waist part 30 contracts in the right-left direction due to the contraction of the elastic members 25 and the elastic members 35 in the right-left direction. In this way, the front-side waist part 20 and the rear-side waist part 30 are formed with a plurality of creases that each rise and fall in the thickness direction. The number of creases per unit region of the front-side waist part 20 that has a greater contractile force is greater than the number of creases per unit region of the rear-side waist part 30 that has a smaller contractile force. Thus, as shown in Fig. 5A, even in the case where there are a large number of holes 50 per unit region, when many creases are formed, there are holes 50 that are hidden with the creases, and the number of holes 50 that can be visually identified when viewing the front-side waist part 20 from the non-skin side becomes few. Further, the holes 50 are also contracted in the right-left direction, together with contracting of the front-side waist part 20 itself in the right-left direction, and it becomes difficult to visually identify the holes 50. In contrast, as shown in Fig. 5B, even when the number of the holes 50 per unit region is few, in the case of viewing the rear-side waist part 30 with less creases from the non-skin side, the possibility that the holes 50 are hidden with the creases decreases, thus the possibility that the holes 50 are hidden with creases in the rear-side waist part 30 with smaller contractile force becomes low. Contraction of the rear-side waist part 30 in the right-left direction becomes smaller compared to contraction of the front-side waist part 20 in the right-left direction, and the contraction degree of the rear-side waist part 30 becomes smaller than the contraction of the holes 50 of the front-side waist part 20 in the right-left direction, thus the holes 50 can be more easily visually identified.

Through-holes had been formed to improve air permeability of disposable diapers before. But, in the natural state of the diaper, such as before wearing the diaper, the front-side waist part 20 and the rear-side waist part 30 were contracted in the right-left direction, and it was difficult to visually identify the holes 50 because of the plurality of creases formed, and it was difficult to visually identify the holes 50 because the holes 50 were contracted in the right-left direction, and it was difficult to confirm from the outside that air permeability has been improved. When an extremely large number of the holes 50 are provided in the front-side waist part 20 and the rear-side waist part 30 so as to be able to visually identify the holes 50, there was a possibility that when such as putting on the diaper the holes 50 may tear and the front-side waist part 20 and the rear-side waist part 30 may be damaged.

In regards to the above point, with the diaper 1, more holes 50 are made in the rear-side waist part 30 that has less creases formed and that has less possibility of the holes 50 being contracted in the right-left direction, than the front-side waist part 20 that has more creases formed due to the contraction being large in the right-left direction and that has a possibility of the holes 50 being contracted in the right-left direction. In this way, the holes 50 are effectively arranged while suppressing a defect such as the front-side waist part 20 and the rear-side waist part 30 being damaged, and a wearer of the diaper 1 or a guardian and the like that is trying to put the diaper 1 on can easily visually identify the holes 50 of the diaper 1 when the diaper 1 is taken out from a package or when the diaper 1 is in a natural state, and it is possible to easily visually identify from the outside that air permeability of the diaper 1 has improved.

Because the diaper 1 includes many holes 50 in the rear-side waist part 30, it is easy to determine which is the front or the rear when trying to put on the diaper 1. By improving air permeability of the rear-side waist part 30, the back of babies and infants when lying facing up can be prevented from becoming stuffy from sweat.

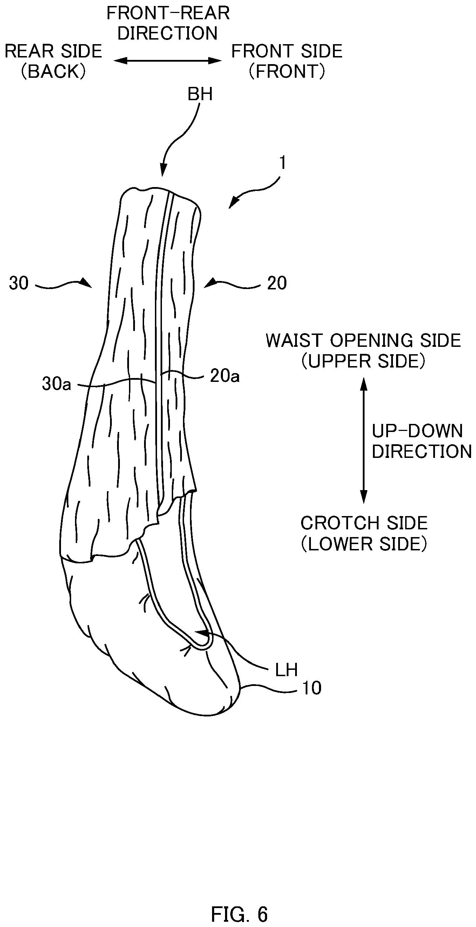

A pull-on diaper 1 may easily be provided with a protruding shape to the front-side waist part 20 side or to the rear-side waist part 30 side because the contractile force of the front-side waist part 20 side and the rear-side waist part 30 are different from each other. Fig. 6 is a schematic side view of the diaper 1 viewed from a one end part side in the right-left direction. Fig. 6 shows the diaper 1 in the natural state, and the diaper 1 in a state that has been taken out of a package is also the same as the above. Because the diaper 1 in the natural state is more contracted in the front-side waist part 20 side that has a greater contractile force, the shape is protruded to the rear-side waist part 30 that has a smaller contractile force. Here, when more holes 50 are included in the rear-side waist part 30 with a small contractile force, the rear-side waist part 30 is pulled with the contraction of the elastic members 25 in the front-side waist part 20, and the rear-side waist part 30 is in a widened state, and the holes 50 of the diaper 1 in the natural state can be more easily visually identified.

The number of the holes 50 in regions 11d that overlap with the absorbent body 11 in the up-down direction in the rear-side waist part 30 is greater than the number of holes 50 in regions that do not overlap with the absorbent body 11 in the up-down direction which is namely regions to the upper side than the regions 11d. Because the absorbent body 11 has relatively higher rigidity than other members, by including more holes 50 in regions 11d that overlap with the absorbent body 11 in the up-down direction, the holes 50 can be easily maintained in an open state, and good air permeability can be easily recognized from the outside.

It is preferable that the holes 50 are included in regions that overlap with the absorbent main body 10 in the up-down direction. When the holes 50 are included to the upper side than the absorbent main body 10, there is a possibility that when a wearer or a person who tries to put on a diaper holds an upper part of the diaper 1 and pulls the diaper up when wearing the diaper the holes 50 may tear in the up-down direction. But by including the holes 50 in regions that overlap with the absorbent main body 10 in the up-down direction, the possibility of the holes 50 tearing may be decreased with the rigidity of the absorbent main body 10.

As shown in Fig. 1A and Fig. 1B, it is preferable to include at least one or more holes 50 in a non-overlapping region 30x which is not overlapped with the front-side waist part 20 in the front-rear direction, of the rear-side waist part 30. The non-overlapping region 30x is a region that covers a buttocks of a wearer when wearing a diaper. As shown in Fig. 1B, when the diaper 1 is viewed from a rear side, not only can the plurality of holes 50 included in the non-overlapping region 30x be visually identified, but as shown in Fig. 1A, when the diaper 1 is viewed from a front side, the holes 50 included in the non-overlapping region 30x can also be visually identified. In this way, because the holes 50 can be visually identified by viewing the diaper 1 from either the front or the rear, good air permeability can easily be recognized from the outside.

In an open and extended state of the diaper shown in Fig. 2, the non-overlapping region 30x is a region in the lower part of the rear-side waist part 30 and has a curved end. It is preferable that an interval Lx between an elastic member 35a (a first elastic member) positioned to the lowest side of the non-overlapping region 30x and an elastic member 35b adjacent to the elastic member 35a to the upper side is greater than an interval Ly between an elastic member 35c (a second elastic member) positioned to the lowest side of the region overlapping the front-side waist part 20 of the rear-side waist part 30 and an elastic member 35d adjacent to the elastic member 35c from the upper side (Lx>Ly) . By arranging the elastic member 35 in this way, the contractile force of the non-overlapping region 30x can be made smaller, and the number of creases formed in the non-overlapping region 30x can be decreased. In this way, the possibility of the holes 50 being covered with the creases in the non-overlapping region 30x can be decreased, and the holes 50 in the non-overlapping region 30x can be visually identified when viewing the diaper 1 from either the front side or the rear side.

It is more preferable that a tension of the elastic member 35a positioned to the lowest side of the non-overlapping region 30x is smaller than a tension of the elastic member 35c positioned to the lowest side of the region overlapping the front-side waist part 20 of the rear-side waist part 30. In this way, the contractile force of the non-overlapping region 30x is made smaller, the number of creases formed in the non-overlapping region 30x is decreased, and, in the non-overlapping region 30x, the possibility that the holes 50 will be covered with creases is decreased, and the holes in the non-overlapping region 30x will be easily visually identified even when viewing the diaper 1 from either the front side or the rear side.

### --- Other Embodiments ===

The embodiment of this invention has been described above, and the above embodiment is to facilitate understanding of this invention, and does not limit this invention in any way. A modification as shown below, for example, may be included.

In the above-described embodiment, the holes 50 were formed in both the front-side waist part 20 and the rear-side waist part 30, but it is not limited to this. The holes 50 may be formed in only either one of the front-side waist part 20 or the rear-side waist part 30. By forming the holes 50 in both the front-side waist part 20 and the rear-side waist part 30, however, the air permeability of the diaper 1 can be further improved.

In the above-described embodiment, more holes 50 were formed in the rear-side waist part 30 with smaller contractile force, but it is not limited to this. The contractile force of the front-side waist part 20 may be made smaller than the rear-side waist part 30, and more holes 50 may be included in the front-side waist part 20 than the rear-side waist part 30. In this way, it becomes easier to visually identify the holes 50 provided in the front-side waist part 20 of the diaper 1 in the natural state such as in a state when taken out from a package, and the front and rear of the diaper 1 can be more easily distinguished. By including more holes 50 in the front-side waist part 20, it becomes easier to let out dampness in babies and infants lying facing down or from discharged urine.

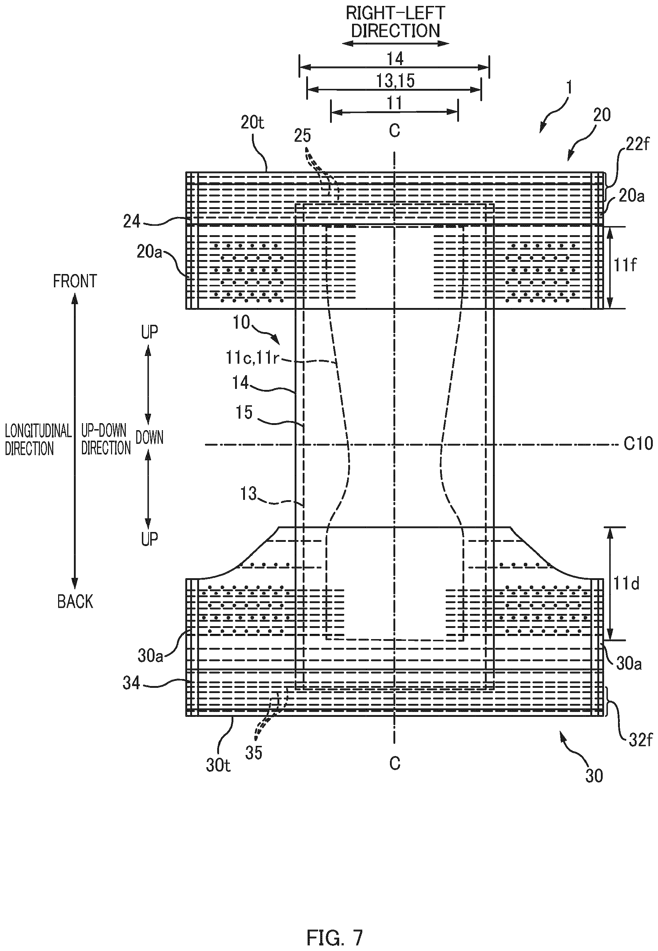

In the above-described embodiment, the length in the up-down direction of the region 11f in which the front-side waist part 20 and the absorbent body 11 overlap with each other and the length in the up-down direction of the region 11d in which the rear-side waist part 30 and the absorbent body 11 overlap with each other are approximately the same, but it is not limited thereto. Fig. 7 is a plan view of a diaper 1 in an open extended state of another embodiment viewed from a skin side. As shown in Fig. 7, by making the length in the up-down direction of the region 11d in which the rear-side waist part 30 with a smaller contractile force and the absorbent body 11 overlap with each other longer than the length in the up-down direction of the region 11f in which the front-side waist part 20 with a greater contractile force and the absorbent body overlap with each other (11d>11f), the holes 50 included in the region which overlaps in the up-down direction with the absorbent body 11 and is relatively higher in rigidity than other sections may easily maintain the open shape state. In this way, the holes 50 of the rear-side waist part 30 can be more easily visually identified from the outside.

As shown in Fig. 7, in the rear-side waist part 30, the holes 50 may be included in the region 11d which overlaps with the absorbent body 11 in the up-down direction, and the holes 50 do not have to be included in the region not overlapping the absorbent body 11 in the up-down direction, namely the upper side of the region 11d. In this way, the holes 50 included in the region 11d which overlaps in the up-down direction with the absorbent body 11 with relatively high rigidity can easily maintain the open state, and good air permeability can be easily visually identified from the outside.

In the above-described embodiment, circular holes 50 are provided in the front-side waist part 20 and the rear-side waist part 30, but it is not limited to this. For example, through holes in the shape of a rectangle, oval, or an arbitrary shape such as a star may be provided. Further, the holes 50 of different shapes and different sizes may be provided to the front-side waist part 20 and to the rear-side waist part 30.

The diaper 1 of the above-described embodiment is subject to be worn by babies and infants, but it is not limited to this, and may be worn by adults. Further, in the above-described embodiment, as an example of an absorbent article, namely a three-piece type disposable diaper 1 is exemplified, but it is not limited to this. An absorbent article may be a so-called two-piece type disposable diaper formed with the front-side waist part 20 and the rear-side waist part 30 in an integrated manner, or may be a tape-type disposable diaper.

### Reference Signs List

1 diaper (absorbent article),
10 absorbent main body, 11 absorbent body,
11c absorbent core, 11r core-wrapping sheet,
11f region, 11d region,
13 top sheet, 14 exterior sheet, 15 back sheet,
20 front-side waist part (waist part, waist part on another/other side),
20a side end part (end part), 20t front-side upper end,
21 skin-side sheet, 22 non-skin side sheet, 22f folded-back section,
24 sheet member, 25 elastic member,
30 rear-side waist part (waist part, waist part on one side),
30a side end part (end part), 30t rear-side upper end,
30x non-overlapping region,
31 skin-side sheet, 32 non-skin side sheet,
32f folded-back section,
34 sheet member, 35 elastic member,
35a elastic member (first elastic member), 35b elastic member,
35c elastic member, (second elastic member), 35d elastic member,
50 hole,
BH waist opening, LH leg opening

## Claims

1. An absorbent article (1) including an up-down direction, a right-left direction, and a front-rear direction, which intersect with each other, comprising:
a front-side waist part (20); and
a rear-side waist part (30),
at least one of the front-side waist part (20) and the rear-side waist part (30) including
an elastic member (25, 35) that expands and contracts in the right-left direction and
holes (50) penetrating in the front-rear direction,
of the front-side waist part (20) and the rear-side waist part (30), a waist part on one side with a smaller contractile force in the right-left direction includes more of the holes (50) than a waist part on another side with a greater contractile force in the right-left direction,
wherein an absorbent body (11) is included, and
a number of the holes (50) in a region, of the waist part on one side, overlapping the absorbent body (11) in the up-down direction is greater than a number of the holes (50) in a region, of the waist part on one side, not overlapping the absorbent body (11) in the up-down direction.

2. An absorbent article (1) according to claim 1, wherein
each of the front-side waist part (20) and the rear-side waist part (30) includes the holes (50).

3. An absorbent article (1) according to claim 1 or 2, wherein
the waist part on one side is the rear-side waist part (30).

4. An absorbent article (1) according to claim 3, wherein
a non-overlapping region (30x), of the rear-side waist part (30), that does not overlap with the front-side waist part (20) in the front-rear direction includes at least one of the holes (50).

5. An absorbent article (1) according to claim 4, wherein
the rear-side waist part (30) includes an overlapping region that overlaps with the front-side waist part (20) in the front-rear direction,
an interval (Lx) between a first lower end elastic member (35a) positioned to a lowest side in the non-overlapping region (30x) and the elastic member (35b) adjacent to the first lower end elastic member in the upper side is greater than an interval (Ly) between a second lower end elastic member (35c) positioned to a lowest side in the overlapping region and the elastic member (35d) adjacent to the second lower end elastic member in the upper side.

6. An absorbent article (1) according to claim 4 or 5, wherein
the rear-side waist part (30) includes an overlapping region that overlaps with the front-side waist part (20) in the front-rear direction,
a tension of a first lower end elastic member (35a) positioned to a lowest side in the non-overlapping region (30x) is smaller than a tension of a second lower end elastic member (35c) positioned to a lowest side in the overlapping region.

7. An absorbent article (1) according to claim 1 or 2, wherein
the waist part on one side is the front-side waist part (20).

8. An absorbent article (1) according to claim 1 to 7, wherein
an absorbent body (11) is included, and
a length of a region (11f) in which the waist part on one side overlaps with the absorbent body (11), in the up-down direction, is longer than a length of a region (11d) in which the waist part to the other side overlaps with the absorbent body (11).

9. An absorbent article (1) according to any one of claims 1 to 8, wherein
the holes (50) are provided in a region, of the waist part on one side, overlapping the absorbent body (11) in the up-down direction, and the holes (50) are not provided in a region, of the waist part on one side, not overlapping the absorbent body (11) in the up-down direction.

10. An absorbent article (1) according to any one of claims 1 to 9, wherein
both end parts (20a) of the front-side waist part (20) in the right-left direction and both end parts (30a) of the rear-side waist part (30) in the right-left direction are joined to each other, and
in a natural state, the front-side waist part (20) and the rear-side waist part (30) are in a protruding shape to the side of the waist part on one side.

## Patentansprüche

1. Absorbierender Artikel (1), der eine Oben-Unten-Richtung, eine Rechts-Links-Richtung und eine Vorn-Hinten-Richtung einschließt, die einander schneiden, der Folgendes umfasst:
ein Vorderseitentaillenteil (20) und
ein Hinterseitentaillenteil (30),
wobei mindestens eines des Vorderseitentaillenteils (20) und des Hinterseitentaillenteils (30) Folgendes einschließt:
ein elastisches Element (25, 35), das sich in der Rechts-Links-Richtung dehnt und zusammenzieht, und
Löcher (50), die in der Vorn-Hinten-Richtung durchgehen,
wobei von dem Vorderseitentaillenteil (20) und dem Hinterseitentaillenteil (30) ein Taillenteil auf einer Seite mit einer kleineren Kontraktionskraft in der Rechts-Links-Richtung mehr der Löcher (50) einschließt als ein Taillenteil auf einer anderen Seite mit einer größeren Kontraktionskraft in der Rechts-Links-Richtung,
wobei ein Saugkörper (11) eingeschlossen ist und
eine Anzahl der Löcher (50) in einem Bereich des Taillenteils auf einer Seite, der mit dem Saugkörper (11) in der Oben-Unten-Richtung überlappt, größer ist als eine Anzahl der Löcher (50) in einem Bereich des Taillenteils auf einer Seite, der nicht mit dem Saugkörper (11) in der Oben-Unten-Richtung überlappt.

2. Absorbierender Artikel (1) nach Anspruch 1, wobei
jedes des Vorderseitentaillenteils (20) und des Hinterseitentaillenteils (30) die Löcher (50) einschließt.

3. Absorbierender Artikel (1) nach Anspruch 1 oder 2, wobei das Taillenteil auf einer Seite das Hinterseitentaillenteil (30) ist.

4. Absorbierender Artikel (1) nach Anspruch 3, wobei
ein nicht-überlappender Bereich (30x) des Hinterseitentaillenteils (30), der nicht mit dem Vorderseitentaillenteil (20) in der Vorn-Hinten-Richtung überlappt, mindestens eines der Löcher (50) einschließt.

5. Absorbierender Artikel (1) nach Anspruch 4, wobei
das Hinterseitentaillenteil (30) einen überlappenden Bereich einschließt, der mit dem Vorderseitentaillenteils (20) in der Vorn-Hinten-Richtung überlappt,
ein Abstand (Lx) zwischen einem ersten elastischen Element am unteren Ende (35a), das an einer untersten Seite in dem nicht-überlappenden Bereich (30x) positioniert ist, und dem elastischen Element (35b) benachbart zu dem ersten elastischen Element am unteren Ende in der oberen Seite größer ist als ein Abstand (Ly) zwischen einem zweiten elastischen Element am unteren Ende (35c), das an einer untersten Seite in dem überlappenden Bereich positioniert ist, und dem elastischen Element (35d) benachbart zu dem zweiten elastischen Element am unteren Ende in der oberen Seite.

6. Absorbierender Artikel (1) nach Anspruch 4 oder 5, wobei
das Hinterseitentaillenteil (30) einen überlappenden Bereich einschließt, der mit dem Vorderseitentaillenteils (20) in der Vorn-Hinten-Richtung überlappt,
eine Spannung eines ersten elastischen Elements am unteren Ende (35a), das an einer untersten Seite in dem nicht-überlappenden Bereich (30x) positioniert ist, kleiner ist als eine Spannung eines zweiten elastischen Elements am unteren Ende (35c), das an einer untersten Seite in dem überlappenden Bereich positioniert ist.

7. Absorbierender Artikel (1) nach Anspruch 1 oder 2, wobei das Taillenteil auf einer Seite das Vorderseitentaillenteil (20) ist.

8. Absorbierender Artikel (1) nach Anspruch 1 bis 7, wobei
ein Saugkörper (11) eingeschlossen ist und
eine Länge eines Bereichs (11f), in dem das Taillenteil auf einer Seite mit dem Saugkörper (11) in der Oben-Unten-Richtung überlappt, länger ist als eine Länge eines Bereichs (11d), in dem das Taillenteil auf der anderen Seite mit dem Saugkörper (11) überlappt.

9. Absorbierender Artikel (1) nach einem der Ansprüche 1 bis 8, wobei
die Löcher (50) in einem Bereich des Taillenteils auf einer Seite, der mit dem Saugkörper (11) in der Oben-Unten-Richtung überlappt, bereitgestellt sind und die Löcher (50) nicht in einem Bereich des Taillenteils auf einer Seite, der nicht mit dem Saugkörper (11) in der Oben-Unten-Richtung überlappt, bereitgestellt sind.

10. Absorbierender Artikel (1) nach einem der Ansprüche 1 bis 9, wobei
beide Endteile (20a) des Vorderseitentaillenteils (20) in der Rechts-Links-Richtung und beide Endteile (30a) des Hinterseitentaillenteils (30) in der Rechts-Links-Richtung miteinander verbunden sind und
in einem natürlichen Zustand das Vorderseitentaillenteil (20) und das Hinterseitentaillenteil (30) in einer herausstehenden Form zu der Seite des Taillenteils auf einer Seite vorliegen.

## Revendications

1. Article absorbant (1) comprenant une direction allant de haut en bas, une direction allant de droite à gauche, et une direction allant de devant à derrière, qui se croisent les unes les autres, comportant :
une partie au niveau de la taille côté avant (20) ; et
une partie au niveau de la taille côté arrière (30),
au moins l'une parmi la partie au niveau de la taille côté avant (20) et la partie au niveau de la taille côté arrière (30) comprenant
un élément élastique (25, 35) qui s'étend et se contracte dans la direction allant de droite à gauche et
des trous (50) pénétrant dans la direction allant de devant à derrière,
parmi la partie au niveau de la taille côté avant (20) et la partie au niveau de la taille côté arrière (30), une partie au niveau de la taille sur un côté ayant une force de traction inférieure dans la direction allant de droite à gauche comprend plus de trous (50) qu'une partie au niveau de la taille sur un autre côté ayant une force de traction supérieure dans la direction allant de droite à gauche,
dans lequel un corps absorbant (11) est inclus, et
un nombre de trous (50) dans une région, de la partie au niveau de la taille sur un côté, chevauchant le corps absorbant (11) dans la direction allant de haut en bas est supérieur à un nombre de trous (50) dans une région, de la partie au niveau de la taille sur un côté, ne chevauchant pas le corps absorbant (11) dans la direction allant de haut en bas.

2. Article absorbant (1) selon la revendication 1, dans lequel
chacune de la partie au niveau de la taille côté avant (20) et de la partie au niveau de la taille côté arrière (30) comprend les trous (50).

3. Article absorbant (1) selon la revendication 1 ou la revendication 2, dans lequel
la partie au niveau de la taille sur un côté est la partie au niveau de la taille côté arrière (30).

4. Article absorbant (1) selon la revendication 3, dans lequel
une région non chevauchante (30x), de la partie au niveau de la taille côté arrière (30), qui ne chevauche pas la partie au niveau de la taille côté avant (20) dans la direction allant de devant à derrière comprend au moins l'un des trous (50).

5. Article absorbant (1) selon la revendication 4, dans lequel
la partie au niveau de la taille côté arrière (30) comprend une région chevauchante qui chevauche la partie au niveau de la taille côté avant (20) dans la direction allant de devant à derrière,
un intervalle (Lx) entre un premier élément élastique d'extrémité inférieure (35a) positionné sur un côté se trouvant le plus en bas dans la région non chevauchante (30x) et l'élément élastique (35b) se trouvant de manière adjacente par rapport au premier élément élastique d'extrémité inférieure dans le côté supérieur est supérieur à un intervalle (Ly) entre un deuxième élément élastique d'extrémité inférieure (35c) positionné sur un côté se trouvant le plus en bas dans la région chevauchante et l'élément élastique (35d) se trouvant de manière adjacente par rapport au deuxième élément élastique d'extrémité inférieure dans le côté supérieur.

6. Article absorbant (1) selon la revendication 4 ou la revendication 5, dans lequel
la partie au niveau de la taille côté arrière (30) comprend une région chevauchante qui chevauche la partie au niveau de la taille côté avant (20) dans la direction allant de devant à derrière,
une tension d'un premier élément élastique d'extrémité inférieure (35a) positionné sur un côté se trouvant le plus en bas dans la région non chevauchante (30x) est inférieure à une tension d'un deuxième élément élastique d'extrémité inférieure (35c) positionné sur un côté se trouvant le plus en bas dans la région chevauchante.

7. Article absorbant (1) selon la revendication 1 ou la revendication 2, dans lequel
la partie au niveau de la taille sur un côté est la partie au niveau de la taille côté avant (20).

8. Article absorbant (1) selon les revendications 1 à 7, dans lequel
un corps absorbant (11) est inclus, et
une longueur d'une région (11f) dans laquelle la partie au niveau de la taille sur un côté chevauche le corps absorbant (11), dans la direction allant de haut en bas, est plus longue qu'une longueur d'une région (11d) dans laquelle la partie au niveau de la taille sur l'autre côté chevauche le corps absorbant (11) .

9. Article absorbant (1) selon l'une quelconque des revendications 1 à 8, dans lequel
les trous (50) sont mis en oeuvre dans une région, de la partie au niveau de la taille sur un côté, chevauchant le corps absorbant (11) dans la direction allant de haut en bas, et les trous (50) ne sont pas mis en oeuvre dans une région, de la partie au niveau de la taille sur un côté, ne chevauchant pas le corps absorbant (11) dans la direction allant de haut en bas.

10. Article absorbant (1) selon l'une quelconque des revendications 1 à 9, dans lequel
les deux parties d'extrémité (20a) de la partie au niveau de la taille côté avant (20) dans la direction allant de droite à gauche et les deux parties d'extrémité (30a) de la partie au niveau de la taille côté arrière (30) dans la direction allant de droite à gauche sont assemblées les unes par rapport aux autres, et
dans un état naturel, la partie au niveau de la taille côté avant (20) et la partie au niveau de la taille côté arrière (30) sont en une forme faisant saillie sur le côté de la partie au niveau de la taille sur un côté.
